# EUROPEAN PATENT APPLICATION

(11) **EP 1 769 843 A2**
(43) Date of publication of application: **04.04.2007**
(21) Application number: 06020607.5
(22) Date of filing: 29.09.2006
(51) Int. Cl.: B01J 13/00, B01F 17/12

(54) **Use of 2,3-Dihydroxynaphthalene-6-Sulfonic Acid Salts as Dispersants**

(30) Priority: 30.09.2005 US 722209 P; 21.09.2006 US 524471 P
(71) Applicant: Air Products and Chemicals, Inc., Allentown, PA 18195-1501 (US)
(72) Inventor: Distefano, Frank Vito, Macungie, PA 18062 (US)
(74) Representative: Kador & Partner

(57) **Abstract**

Stable dispersions of nanoparticles and microparticles in liquids and method for their preparation are disclosed. The dispersions comprise about 0.1 wt% to about 25 wt% of a 2,3-dihydroxynaphthalene-6-sulfonic acid salt or mixture of salts of 2,3-dihydroxynaphthalene-6-sulfonic acid; about 1 wt% to about 90 wt% of the particles; and about 10 wt% to about 90 wt% of the liquid.

## Description

### CROSS-REFERENCE TO RELATED APPLICATIONS

This Application claims the benefit of Provisional Application No. 60/722,209, filed on September 30, 2005. The disclosure of the Provisional Application is hereby incorporated by reference.

### BACKGROUND OF THE INVENTION

The invention relates to dispersions. In particular, the invention relates to stable dispersions of nanoparticles and microparticles in liquids and to methods for their preparation.

Nanoparticles of Group IIIA metal oxides, specifically, those of aluminum and indium have important commercial applications. Nano alumina is of interest for scratch resistant coatings and heat transfer fluids. Additionally, aluminum metal nanoparticles that have been passivated with a thin layer of aluminum oxide are of use in the development of energetic materials. Indium tin oxide (ITO) nanoparticles have applications in clear conductive coatings, in heat management layers, and in static charge dissipation. Other applications of metal oxide nanoparticles and/or nanoparticles that have a metal oxide surface include magnetic materials, heterogeneous catalysts, toner compositions, and ceramics.

In order to supply nanoparticles and/or microparticles as easy to use dispersion master batches or in fully formulated compositions, the particles must be dispersed in various liquids and polymeric matrices. The quality of the dispersion must support its intended use. For example, the presence of color is unacceptable in many applications, including inks and coatings. In addition, the dispersion is preferably stable so it does not have to be prepared immediately before use, but can be stored after preparation.

Currently, many nanoparticle dispersions are prepared by functionalizing the surface of the particles with materials such as silanes. This approach uses expensive silanes and requires additional processing steps. Alternatively, ionic dispersants that rely upon electrostatic attraction for anchoring to the particle surface are used. Below the isoelectric point, where the nanoparticle is inherently cationic, an anionic dispersant is required to achieve surface anchorage. Above the isoelectric point, where the particle is inherently anionic, a cationic dispersant is required. Consequently, the resulting dispersion can not tolerate a wide pH range. In addition, many materials used in coatings, inks, are anionic and not compatible with cationic materials. Thus, a need exists for stable dispersions of nanoparticles and/or microparticles particles of metal oxides and/or particles that have an metal oxide surface that do not have these problems, and to methods for preparing these dispersions.

### BRIEF SUMMARY OF THE INVENTION

The invention comprises a composition comprising a dispersion of particles in at least one liquid (e.g., at least one polar liquid). The composition comprises:
a) about 0.1 wt% to about 25 wt%, based on the total weight of the dispersion, of at least one dispersant the formula: 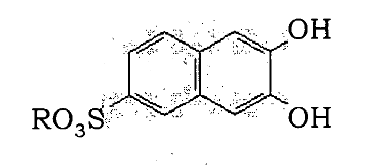 in which R comprises a cation selected from the group consisting of Na⁺, Li⁺, K⁺, and NR'₄⁺, in which each R' is independently -H, -CH₃, -CH₂-CH₃, or -CH₂-CH₂-OH;
b) about 1 wt% to about 90 wt%, based on the total weight of the dispersion, of particles of at least one member selected from the group consisting of metal particles, metal oxide particles, particles having a metal oxide surface, and mixtures thereof, in which the particles have a particle size of about 1 nm to about 2000 nm;
c) about 10 wt% to about 90 wt%, based on the total weight of the dispersion, of at least one liquid selected from the group consisting of water, ethylene glycol, propylene glycol, glycerin, glycol mono-ethers of the formula R"OCH₂CH₂OH, in which R" is an alkyl group of one to four carbon atoms, and mixtures thereof; and
d) optionally about 1 wt% to about 99 wt%, based upon the total weight of the dispersion, comprising at least one member selected from the group consisting of water-borne polymers such as emulsion polymers, aqueous polymer dispersions, aqueous polymer colloids, and aqueous polymer solutions. These water-borne polymers may comprise at least one of urethane, acrylic, styrene-acrylic, elastomers (e.g., styrene-butadiene copolymer, natural rubber, polychloroprenes, butadiene-acyrilonitrile copolymer, and polyisoprene, among others) siloxane, vinyl acetate, vinyl chloride and among other polymers

In one aspect, the particles are nanoparticles, having an average diameter of about 1 nm to about 100 nm. In another aspect, the invention is a method for preparing the dispersion by dispersing the particles in the liquid containing the dispersant.

### DETAILED DESCRIPTION OF THE INVENTION

Unless the context indicates otherwise, in the specification and claims, the terms particles, metal oxide particles, particles having a metal oxide surface, dispersant, liquid, cation, and similar terms also include mixtures of such materials. Unless otherwise specified, all percentages are percentages by weight and all temperatures are in degrees Centigrade (degrees Celsius).

In one aspect the invention comprises a dispersion of particles having a particle size of about 1 nm to about 2000 nm in liquid. The particles are selected from the group consisting of metal particles, metal oxide particles, particles having a metal oxide surface, and mixtures thereof. The dispersion comprises the dispersant, the particles, and liquid.

The dispersant comprises a salt or mixture of salts of 2,3-dihydroxynaphthalene-6-sulfonic acid having the following formula:

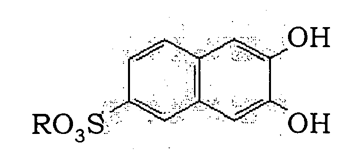

R comprises a cation selected from the group consisting of Na⁺, Li⁺, K⁺, and NR'₄⁺ in which each R' is independently -H, -CH₃, -CH₂-CH₃, -CH₂-CH₂-OH. These compounds are useful as a dispersants for particles comprising at least one member selected from the group consisting of metal oxide particles, particles having a metal oxide surface, and mixtures thereof in water and other polar liquids. A useful dispersant comprises the sodium salt (R = Na⁺), 2,3-dihydroxynaphthalene-6-sulfonic acid sodium salt. The sodium salt, which is used as a coupler in diazo photosensitive paper, is commercially available under the trade name "Dihydroxy R Salt" or "DHR".

The dispersion comprises microparticles and/or nanoparticles. Nanoparticles generally refers to particles that have an average diameter of about 100 nm or less, typically between about 100 nm and about 1 nm. Nanoparticles have an intermediate size between individual atoms and macroscopic bulk solids. Because of their small size, the physical and chemical properties of nanoparticles, especially those of nanoparticles smaller than about 50 nm, may differ measurably from those of the bulk material. Microparticles are larger than nanoparticles. They have an average diameter of about 100 nm (0.1 micron) to about 100 microns. The dispersion typically comprises particles that have an average diameter of about 2000 nm or less, typically an average diameter of about 1 nm to about 2000 nm.

Particle size refers to the size of the particles determined by the BET (Brunauer, Emmet, Teller) method. This method, which involves adsorbing a monolayer of liquid nitrogen onto the surface of a mass of particles, then measuring the amount of nitrogen released when that monolayer is vaporized, is well known to those skilled in the art. The particle size measured for the particles in the dispersion, which is measured by other methods, may be larger than the particle size determined by the BET method because of aggregation of the primary nanoparticles into aggregates. As discussed below, the particle size measured for the particles in the dispersion is a measure of the ability of the dispersing agent to produce a dispersion.

The particles comprise at least one member selected from the group consisting of metal particles, metal oxide particles, particles having a metal oxide surface, and mixtures thereof. Although the metal oxide particles may be particles of any metal oxide that forms the dispersion, typical metal oxides include alumina (Al₂O₃), indium tin oxide (a mixture of In₂O₃ and SnO₂), zirconia (ZrO₂), titania (TiO₂), iron oxide (Fe₂O₃), ceria (CeO₂), and mixtures thereof. More typically, the metal oxide comprises alumina or indium tin oxide. The metal oxide particles may be doped with other materials. Typical particles having a metal oxide surface include aluminum metal particles with a surface layer of aluminum oxide. Examples of metal particles comprise at least one member selected from the group consisting of alminum, copper, silver, tin, indium, antimony, iron, or zirconium.

The liquid may be any liquid (e.g., a polar liquid) in which the dispersion may be formed. Typical liquids comprise at least one member selected from the group consisting of water, ethylene glycol, glycerin, propylene glycol, ethylene glycol mono-ethers, and mixtures thereof. Typical ethylene glycol mono-ethers are compounds of the structure R"OCH₂CH₂OH, in which R" comprises an alkyl group of one to four carbon atoms, such as methyl, ethyl, *n*-propyl, or *n*-butyl. Common ethylene glycol mono-ethers include 2-methoxyethanol (methyl CELLOSOLVE®) and 2-butoxyethanol (butyl CELLOSOLVE®).

Typically, the dispersion comprises about 0.1 wt% to about 25 wt% of the dispersant, about 1 wt% to about 90 wt% of the particles, and about 10 wt% to about 90 wt% of the liquid, based on the total weight of the dispersion. More typically the dispersion comprises about 0.1 wt% to about 10 wt% of the dispersant, about 5 wt% to about 80 wt% of the particles, and about 5 wt% to about 80 wt% of the liquid, based on the total weight of the dispersion. Most typically the dispersion comprises about 0.1 wt% to about 5 wt% of the dispersant, about 10 wt% to about 70 wt% of the particles, and about 25 wt% to about 75 wt% of the liquid, based on the total weight of the dispersion. Typically, the dispersant, the particles, and the liquid together make up at least about 95 wt%, more typically at least about 98 wt% up to about 100 wt%, of the dispersion. The dispersion may consists essentially of the particles, the dispersant, and the liquid, or the dispersion may comprise other ingredients that are commonly used in dispersions used in the inks, coatings, and/or adhesives, such as, for example, other dispersants; surfactants, such as, for example, nonionic and anionic surfactants; defoamers; and wetting agents.

Typically, the salt or mixture of salts of 2,3-dihydroxynaphthalene-6-sulfonic acid is the only dispersant necessary. However, about 0.1 to about 10 wt% of other dispersants, such as Tiron (the disodium salt monohydrate of 4-5-dihydroxy-1,3-benzenedisulfonic acid), naphthalene sulfonic acid salts, polyacrylic acid salts, citric acid salts, poly(styrene-co-maleic anhydride) salts, polyoxyethylenated long-cain amines, polyoxyethylenated alkyphenols, polyoxyethylenated alcohols, polyoxyethylenated carboxylic acids, polyoxyethylenated sorbitol esters, polyoxyethylenated alkanolamides, long-chain carboxylic acid esters, poly(ethylene oxide-co-propylene oxide) and sulfonated, sulfated, phosphated or phosphonated derivatives of the above; the class of materials known as polymeric dispersing agents which comprise certain polyacrylates, polyesters, polyamides, maleic acid/vinyl polyether copolymer, styrene-maleic acid copolymers, polyurethanes, polyimides, polyethers, polysilicones, as well as amine, alcohol, acid, ester and other functionalized derivatives of the foregoing and copolymers of the same, among others, may be present.

Surfactants may be present at levels of about 0.1 to about 10.0 wt%. Nonionic surfactants are well know to those skilled in the art and can comprise at least one ethoxylates of alkyl phenols containing from about 8 to 18 carbon atoms in a straight-or branched chain alkyl group, such as *t*-octyl phenol and *t*-nonyl phenol with about 5 to 30 moles of ethylene oxide; and ethoxylates of primary alcohols containing about 8 to 18 carbon atoms in a straight or branched chain configuration with about 5 to 30 moles of ethylene oxide, for example, lauryl or myristyl alcohol condensed with about 16 moles of ethylene oxide. Anionic surfactants are well known to those skilled in the art. Anionic surfactants are salts, especially water soluble salts in which the cation comprise sodium, potassium, ammonium, or substituted ammonium, such as the cations of ethanol amine, diethanol amine, and triethanol amine salts and in which the surfactant portion is negatively charged. These surfactants can comprise at least one C₈-C₂₂ alkyl sulfates, alkyl sulfonates, alkyl sulfosuccinates, and alkylbenzene sulfonates, such as linear alkylbenzene sulfates and sulfonates; sulfates of ethoxylated C₈-C₂₂ alkyl alcohols in which the alkyl group contains about 10 to about 22 and the polyoxyethylene chain contains about 0.5 to about 22 moles of ethylene oxide alkyl alcohol; and phosphates of alkyl alcohols, ethoxylated alkyl alcohols, and ethoxylated alkyl phenols.

Defoamers may be present at levels of about 0.01 to about 3.0 wt %. Defoamers can comprise at least one of silicones such as polyether modified dimethylsiloxanes, for example BYK 307 and BYK 333 (Byk Chemie, Wallingford, CT, USA), and acetylinic diols such as those sold under the SURFYNOL® trademark (Air Products and Chemicals, Allentown, PA, USA). Wetting agents may be present at levels of about 0.1 to about 10.0 wt%. Wetting agents can comprise at least one of sodium dioctylsulfosuccinate and acetylinic diols such as those sold under the DYNOL® trademark (Air Products and Chemicals, Allentown, PA, USA).

The particles can form a stable dispersion in the liquid. That is, the resulting dispersion does not exhibit separation of components, a dramatic increase in viscosity, and/or flocculation of the particles within 24 hours. Typically, the dispersion is stable for at least seven days. This allows master batches to be prepared and stored until needed.

In another aspect, the invention comprises a method for preparing the stable dispersion. The method comprises dispersing the particles in the liquid containing the dispersant. The dispersant is dissolved in the polar liquid and the pH adjusted, if necessary. For example, if the dispersion is ultimately to be used in a formulation that is typically in the range of pH 8-9, such as many inks or coatings, the dispersant solution can be adjusted to this pH range by addition of about 10% aqueous sodium hydroxide or an amine such as AMP-95 (2-amino-2-methyl-1-propanol). Then the particles are dispersed in the liquid containing the dispersant. The particles may be dispersed using equipment typically used in the ink, coating, and/or adhesive industries. This equipment is well known to those skilled in the art, and includes, for example, ball mills, stirred bead mills, homogenizers, roll mills, and ultrasonication baths.

The metal oxide particle dispersions may be supplied as a "solution" (*i.e.,* low solids dispersion) or a very high solids (>70%) paste. Typically a very high solids paste is useful in applications where the total liquid content of the final coating, such as in ink or adhesive applications, must be minimized.

### INDUSTRIAL APPLICABILITY

The dispersions of the invention contain high levels of particles. They may be used as master batches in the preparation of, for example, inks, coatings, and adhesives with enhanced mechanical, chemical, electrical, optical or magnetic properties. Because the dispersion is stable, it does not have to be prepared immediately before use. Large amounts can be prepared, which can be stored for future use.

The advantageous properties of this invention can be observed by reference to the following examples, which illustrate but do not limit the invention.

### EXAMPLES

Glossary
- Alumina A: Spherical gamma alumina (BET particle size - 20nm)
- Alumina B: Spherical gamma alumina (BET particle size - 30nm)

- Alumina C: Spherical 70:30 gamma/delta alumina (BET particle size - 47nm)
- AMP-95: 2-Amino-2-methyl-1-propanol
- DHR Salt: 2,3-Dihydroxy-6-naphthalene sulfonic acid sodium salt
- Tiron: Disodium salt monohydrate of 4-5-dihydroxy-1,3-benzenedisulfonic acid

### Examples 1-14

These examples compare the dispersant properties of DHR Salt in water with other dispersants.

Aqueous nanoparticle dispersions were prepared and evaluated by the following procedure. The dispersant was added to water, and the pH of the resulting solution adjusted to 8.0-9.0 with 10% aqueous sodium hydroxide. Alumina particles were added and, if necessary, the pH readjusted to 8.0-9.0. The resulting mixture was placed in an ultrasonication bath (Branson Model 3510, Branson Ultrasonics, Danbury, CT, USA) for 1 hr at 50°C. It was shaken by hand after 20 min and after 40 min of sonication.

The resulting dispersion was evaluated by the following procedures:

### Sediment

The dispersion (1 g) was added to 9 g of water at pH 8.0 and the resulting mixture centrifuged at 3750 rpm for 30 min (Beckman Instruments Centrifuge CS-6). The upper layer was decanted. The sediment was dried and weighed.

### Sedimentation at High Dilution

One drop of the resulting dispersion was added to 10 g of water at pH 8.0 and shaken. The resulting mixture was allowed to settle over several days and the sedimentation rate was observed.

The results are shown in Table 1.

**Table 1**

| Dispersion of 20% Nano Alumina A in Water | | | | |
|---|---|---|---|---|
| Ex #. | Dispersant (2%) | Sediment (%) | Visual Observation | Sedimentation at high dilution^{a} |
| 1 | DHR Salt | 53.0 | Low viscosity, white | slight sediment |
| 2 | None | 100 | Solid paste | NA |
| 3 | Tiron | 51.3 | Low viscosity, white | very slight sediment |
| 4 | 4,5-Dihydroxynaphthalene-2,7-disulfonic acid, Na⁺ salt | 65.0 | Very viscous, flows when shaken, brownish red | slight sediment |
| 5 | Sulfonated lignosulfonate | 100 | Solid paste | NA |
| 6 | Tetramethylammmonium hydroxide | 100 | Solid paste | NA |
| 7 | Tetraethylammonium hydroxide | 100 | Solid paste | NA |
| 8 | Sulfosalicylic Acid | 64.3 | Very viscous, white | slightly more settling than Tiron |
| 9 | Sulfoisophthalic Acid | 100 | Solid paste, white | completely settled |
| 10 | 4-Methylcatechol sulfonic acid, Na⁺ salt | 53.9 | Low viscosity, brown | very slightly settled |
| 11 | Pyrogallol | 49.8 | Low viscosity, black | very slightly settled |
| 12 | 3,4 Dihydroxybenzoic Acid | 53.9 | Low viscosity, brown | very slightly settled |
| 13 | Gallic Acid | 53.6 | Low viscosity, brown | slightly more settling than Tiron |
| 14 | 2,3 dihydroxybenzoic acid | 40.3 | Low viscosity, green | slightly less settling than Tiron |

| | | | | |
|---|---|---|---|---|
| ^{a}NA = not applicable | | | | |

DHR Salt compares favorably with Tiron in its ability to disperse particles. In contrast, 4,5-dihydroxynaphthalene-2,7-disulfonic acid disodium salt, does not perform well as a dispersant, producing a very viscous, brownish-red dispersion. Sulfonated lignosulfonate, tetramethylammmonium hydroxide, and tetraethylammmonium hydroxide, commercial dispersants known to disperse alumina in aqueous media, were unsuitable for use with alumina particles. The other materials tested (Examples 8-14), did not produce low viscosity dispersions and/or gave highly colored products.

### Example 15

This example demonstrates that DHR salt is an effective dispersant at low pH.

A dispersion was prepared as described in Example 1, except that the dispersion contained 50% of the alumina B nanoparticles in water and the pH was not adjusted with 10% aqueous sodium hydroxide. The pH of the dispersion was 4.8. The viscosity of the dispersion, measured at 20 rpm with a Brookfield Model DVII+Viscometer (Brookfield Engineering, Middleboro, MA), was 30 cps.

### Examples 16-19

These examples shows the dispersant properties of 2,3-dihydroxynaphthalene-6-sulfonic acid sodium salt in ethylene glycol and in mixtures of ethylene glycol with other liquids.

The procedure of Example 1 was repeated, except that the dispersant was dissolved in ethylene glycol instead of water and dispersions were prepared by sonicating for 2 hr at 65°C. AMP-95 was added to adjust the pH to 8. Viscosity was measured as described in Example 15. Particle size and zeta potential were measured as described below. The results are give in Table 2.

### Particle Size and Zeta Potentia

Samples were diluted to 0.1 % solids in the same liquid used to make the dispersion. Particle size and zeta potential were determined using a Malvern Nanosizer (Malvern, Worcestershire, UK) and Malvern ZETASIZER® (Malvern, Worcestershire, UK).

**Table 2**

| Dispersions in Ethylene Glycol | | | | | | | | |
|---|---|---|---|---|---|---|---|---|
| Ex. | Alumina % (type) | DHR Salt | Tiron | AMP-95 | Ethylene Glycol | Viscosity 20 rpm (Cps) | Particle size (nm) | Zeta potential (mv) |
| 16 | 58.3 (A) | | 1.9 | 1 | 38.8 | 1150 | 130 | -84 |
| 17 | 29.3 (A) | 1.4 | | 0.7 | 68.6 | Fluid dispersion | 118 | -87 |
| 18 | 30 (A) | 2 | | 1 | 67 | Fluid dispersion | 113 | -76 |
| 19 | 58.8 (A) | 2 | | 1 | 38.2 | Fluid dispersion | 114 | -82 |
| 20 | 57.7 (C) | 1.9 | | 1.9 | 9.6^{a} | 33 | | |
| 21 | 48.5 (B) | 1.9 | | 1 | 24.3^{b} | 530 | | |

| | | | | | | | | |
|---|---|---|---|---|---|---|---|---|
| ^{a}Plus 28.9 wt% water ^{b}Plus 24.3 wt% glycerin | | | | | | | | |

Both particle size and zeta potential show dispersion quality. The nanopowders received from suppliers are typically comprised of aggregates containing hundreds of thousands of primary nanoparticles. These aggregates are several microns in diameter. The ability to disperse these aggregates into much smaller clusters is a measure of dispersion efficacy. Zeta potential shows dispersion stability. The zeta potential measures the charge on the particles surface. A high negative or positive zeta potential means that the particles will repel each other, rather than being attracted and flocculating. Because inks, coatings and adhesives are comprised of anionic ingredients, an anionic nanodispersion is typically employed over a cationic one. Dispersions prepared with DHR salt (Examples 17-19) compare very favorably with those made with the Tiron control (Example 16) in both particle size and surface charge.

### Example 20-21

These examples show the dispersant properties of 2,3-dihydroxynaphthalene-6-sulfonic acid sodium salt in an ethylene glycol/water and ethylene glycol/glycerin mixtures, respectively.

The dispersion of Example 20 was prepared as in Example 16. It contained 57.7 parts of Alumina C, 9.6 parts of ethylene glycol, 28.9 parts of water, 1.9 parts of DHR Salt, and 1.9 parts of AMP-95. The dispersion had a viscosity of 33 cps at 20 rpm.

The dispersion of Example 21 was prepared as in Example 16. It contained 48.5 parts of Alumina B, 24.3 parts of ethylene glycol, 24.3 parts of glycerin, 1.9 parts of DHR Salt, and 1 part of AMP-95. The dispersion had a viscosity of 530 cps at 20 rpm.

## Claims

1. A composition comprising:
a) about 0.1 wt% to about 25 wt%, based on the total weight of the dispersion, of a dispersant the formula: 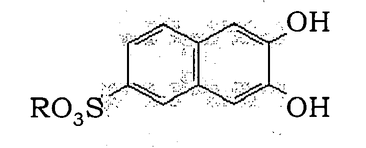 in which R is a cation selected from the group consisting of Na⁺, Li⁺, K⁺, and NR'₄⁺, in which each R' is independently -H, -CH₃, -CH₂-CH₃, or -CH₂-CH₂-OH;
b) about 1 wt% to about 90 wt%, based on the total weight of the dispersion, of particles selected from the group consisting of metal oxide particles, particles having a metal oxide surface, metal particles and mixtures thereof,
in which the particles have a particle size of about 1 nm to about 2000 nm; and
c) about 10 wt% to about 90 wt%, based on the total weight of the dispersion, of a liquid selected from the group consisting of water, ethylene glycol, propylene glycol, glycerin, glycol mono-ethers of the formula
R"OCH₂CH₂OH,
in which R" is an alkyl group of one to four carbon atoms, and mixtures thereof;
in which the particles are dispersed in the polar liquid.

2. The composition of claim 1 in which the dispersant is 2,3-dihydroxy-6-naphthalene sulfonic acid sodium salt.

3. The composition of claim 1 in which the polar liquid comprises at least one member selected from the group consisting of water, ethylene glycol, propylene glycol, glycerin, and mixtures thereof.

4. The composition of claim 1 in which the particles have a particle size of about 1 nm to about 100 nm.

5. The composition of claim 1 in which the composition comprises about 0.1 wt% to about 10 wt% of the dispersant, about 5 wt% to about 80 wt% of the particles, and about 5 wt% to about 80 wt% of the polar liquid.

6. The composition of claim 5 in which the composition comprises about 0.1 wt% to about 5 wt% of the dispersant, about 10 wt% to about 70 wt% of the particles, and about 25 wt% to about 75 wt% of the polar liquid.

7. The composition of claim 5 in which the particles comprise at least one member selected from the group consisting of alumina particles, indium tin oxide particles, zirconia particles, titania particles, iron oxide particles, ceria particles, aluminum metal particles with a surface layer of aluminum oxide, and mixtures thereof.

8. The composition of claim 7 in which the liquid comprises at least one member selected from the group consisting of water, ethylene glycol, propylene glycol, glycerin, and mixtures thereof.

9. The composition of claim 8 in which the dispersant comprises 2,3-dihydroxy-6-naphthalene sulfonic acid sodium salt.

10. The composition of claim 9 in which the particles have a particle size of about 0.1 micron to about 100 microns.

11. The composition of claim 9 in which the particles have a particle size of about 1 nm to about 100 nm.

12. The composition of claim 11 in which the composition comprises about 0.1 wt% to about 5 wt% of the dispersant, about 10 wt% to about 70 wt% of the particles, and about 25 wt% to about 75 wt% of the polar liquid.

13. The composition of claim 12 in which the particles are alumina particles.

14. The composition of claim 13 in which the particles have a particle size of about 1 nm to about 50 nm.

15. The composition of claim 12 in which the particles are indium tin oxide particles.

16. The composition of claim 15 in which the particles have a particle size of about 1 nm to about 50 nm.

17. The composition of claim 14 in which the liquid comprises water.

18. The composition of claim 1 further comprising about 0.1 to about 10 wt%, based upon the total weight of the composition, of at least one member selected from the group consisting of a disodium salt monohydrate of 4-5-dihydroxy-1,3-benzenedisulfonic acid, naphthalene sulfonic acid salts, polyacrylic acid salts, citric acid salts, poly(styrene-co-maleic anhydride) salts, polyoxyethylenated long-cain amines, polyoxyethylenated alkyphenols, polyoxyethylenated alcohols, polyoxyethylenated carboxylic acids, polyoxyethylenated sorbitol esters, polyoxyethylenated alkanolamides, long-chain carboxylic acid esters, poly(ethylene oxide-co-propylene oxide) and sulfonated, sulfated, phosphated or phosphonated derivatives of the foregoing; polyacrylates, polyesters, polyamides, maleic acid/vinyl polyether copolymer, styrene-maleic acid copolymers, polyurethanes, polyimides, polyethers, polysilicones, as well as amine, alcohol, acid, ester and other functionalized derivatives of the foregoing and copolymers of the same.

19. The composition of claim 1 further comprising about 1 wt% to about 99 wt%, based upon the total weight of the dispersion, of at least one member selected from the group consisting of emulsion polymers, aqueous polymer dispersions, aqueous polymer colloids, aqueous polymer solutions.

20. The composition of claim 19 wherein the polymers comprise at least one member selected from the group consisting of urethanes, acrylics, styrene-acrylics, elastomers, styrene-butadiene copolymers, natural rubbers, polychloroprenes, butadiene-acyrilonitrile copolymers, polyisoprene, siloxanes, vinyl acetates, and vinyl chlorides.

21. A method for forming a stable dispersion of particles in a liquid, the method comprising the step of dispersing of the particles in the liquid comprising a dispersant, in which:
the resulting stable dispersion comprises about 0.1 wt% to about 25 wt% of the dispersant, about 1 wt% to about 90 wt% of the particles, and about 10 wt% to about 90 wt% of a polar liquid, based on the total weight of the dispersion;
the dispersant is a compound of the formula: 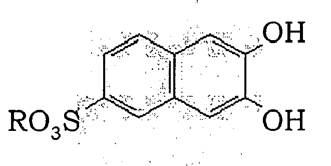
in which R is a cation selected from the group consisting of Na⁺, Li⁺, K⁺, and NR'₄⁺, in which each R' is independently -H, -CH₃, -CH₂-CH₃, or -CH₂-CH₂-OH; the particles are selected from the group consisting of metal oxide particles, particles having a metal oxide surface, and mixtures thereof, in which the particles have a particle size of about 1 nm to about 2000 nm; and
the liquid selected from the group consisting of water, ethylene glycol, propylene glycol, glycerin, glycol mono-ethers of the formula R"OCH₂CH₂OH, in which R" is an alkyl group of one to four carbon atoms, and mixtures thereof.

22. The method of claim 21 in which the dispersant comprises 2,3-dihydroxy-6-naphthalene sulfonic acid sodium salt.

23. The method of claim 22 in which the dispersion comprises about 0.1 wt% to about 10 wt% of the dispersant, about 5 wt% to about 80 wt% of the particles, and about 5 wt% to about 80 wt% of the polar liquid.

24. The method of claim 23 in which the particles comprise at least one member selected from the group consisting of alumina particles, indium tin oxide particles, zirconia particles, titania particles, iron oxide particles, ceria particles, aluminum metal particles with a surface layer of aluminum oxide, and mixtures thereof.

25. The method of claim 24 in which the liquid comprises at least one member selected from the group consisting of water, ethylene glycol, propylene glycol, glycerin, and mixtures thereof.

26. The method of claim 25 in which the composition comprises about 0.1 wt% to about 5 wt% of the dispersant, about 10 wt% to about 70 wt% of the particles, and about 25 wt% to about 75 wt% of the polar liquid.

27. The method of claim 26 the particles have a particle size of about 0.1 micron to about 100 microns.

28. The method of claim 26 in which the particles have a particle size of about 1 nm to about 100 nm.

29. The method of claim 27 in which the particles comprise alumina particles or indium tin oxide particles.

30. The method of claim 29 in which the particles have a particle size of about 1 nm to about 50 nm.
